(19) Europäisches Patentamt
European Patent Office
Office européen des brevets

(11) Publication number: 0 111 354 B1

(12) **EUROPEAN PATENT SPECIFICATION**

(45) Date of publication of patent specification: **30.12.86**

(51) Int. Cl.$^4$: **C 07 C 143/34,** C 11 D 1/22, E 21 B 43/22, B 01 F 17/12

(21) Application number: **83201647.1**

(22) Date of filing: **18.11.83**

# (54) Alkylxylene sulphonate compounds, their preparation and use.

(30) Priority: **13.12.82 GB 8235499**

(43) Date of publication of application: **20.06.84 Bulletin 84/25**

(45) Publication of the grant of the patent: **30.12.86 Bulletin 86/52**

(84) Designated Contracting States: **BE DE FR IT NL**

(56) References cited:
**DE-C- 69 072**
**US-A-2 467 131**
**US-A-3 933 201**

**JOURNAL OF THE AMERICAN OIL CHEMISTS' SOCIETY, vol. 55, May 1978, pages 505-512, Champaign, US, P.H. DOE et al.: "Surfactants for producing low interfacial tensions: II. Linear alkylbenzenesulfonates with additional alkyl substituents"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Bolsman, Theodorus Antonius B. M.**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 111 354 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

This application relates to alkylxylene sulphonate compounds, to a process for preparing them, to their use as a surfactant, to detergent compositions containing them, and to a method for displacing an oil within a subterranean reservoir.

Alkyl *ortho*-xylene sulphonate compounds are known and have been recommended for use in surfactant or detergent compositions, and particularly as chemicals for use in enhanced oil recovery techniques, for example in compositions such as those advertised by member companies of the Royal Dutch/Shell group of companies under the registered trade mark "ENORDET LXS". Such compositions may often be more cost effective than other conventional compounds such as petroleum sulphonates due to superior chemical and thermal stability, but their application tends to be limited to use in reservoirs of relatively low salinity because of limitations of solubility and salt tolerance in water.

Doe *et al* in J. Amer. Oil Chem. Soc., *55* (1978) 505—512 describe various mono-, di- and tri-alkyl benzene sulphonates and their surfactant properties, with a view to their potential efficiency in enhanced oil recovery techniques. One trialkyl benzene sulphonate compound disclosed is derived from *meta*-xylene, by Friedel-Crafts acylation using octanoyl chloride to give a phenyl ketone which was in turn reacted with n-butyl magnesium bromide, the resulting tertiary alcohol being catalytically reduced to give a trialkyl benzene product which was sulphonated, the final product being sodium 2,4-dimethyl-5-(1-butyloctyl)benzene sulphonate. However on Page 510 it is stated in the general discussion on the trialkyl benzene sulphonate surfactants studied that maximum molecular weight efficiency is obtained with dialkylbenzene sulphonates, and hence by implication that those in the art should look no further at compounds such as 2,4-dimethyl-5-(1-butyloctyl)benzene sulphonates.

US patent specification 2,467,131 discloses certain alkyl-metaxylene sulphonates and their use as detergent, but is completely silent about the use of these compounds for displacing oil within a subterranean reservoir and then injecting water.

It has now surprisingly been found that certain novel alkylxylene sulphonate compounds derived from *meta*- and *para*-xylene are potentially useful in enhanced oil-recovery techniques in reservoirs of relatively higher salinities than those to which the conventional alkyl *ortho*-xylene sulphonate compounds are limited.

According to the present invention therefore there are provided alkylxylene sulphonate compounds of formula

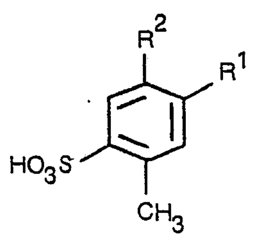

(I)

wherein one of $R^1$ and $R^2$ is a methyl group, the other being a $C_{6-20}$ alkyl group of formula

$$-\underset{\displaystyle (CH_2)_{n'}H}{\underset{|}{C}H}(CH_2)_nCH_3 \qquad (II)$$

wherein n' is 0, 1, 2 or 3 and (n + n') is a number in the range 4 to 18; with the proviso that when both methyl groups are in the meta-position in relation to each other and when n' is 1, n is not equal to a number from 7 to 10; or in the form of a metal salt or an ammonium salt thereof.

Suitable metal salts may include for example sodium, potassium, calcium, magnesium or iron salts. Suitable ammonium salts include those derived from ammonia and from primary, secondary or tertiary amines, for example amines bearing one, two or three $C_{1-6}$ alkyl or hydroxyalkyl moieties, e.g. triethanolamine. Alternative ammonium salts are quaternary ammonium salts such as tetramethyl ammonium salts. Sodium salts have been found to have very good properties.

Most preferred compounds are those derived from *para*-xylene, i.e. those wherein $R^2$ in formula (I) represents a methyl group.

Preferred compounds of the invention are derived from alkylxylenes prepared by alkylation of an appropriate xylene with one or more linear alpha olefins, in which case in the compounds of the invention n' will be 1, 2 or 3.

Chemicals for use in enhanced oil-recovery techniques under saline conditions should desirably have as high a midpoint salinity (MPS), as high a solubilization parameter (SP) and as large a salinity window (SW) as possible. It will be appreciated by those skilled in the art that choosing such chemicals generally involves reaching as advantageous a compromise of MPS, SP and SW properties as possible. In the compounds of the invention the alkyl group is preferably a $C_{8-18}$ alkyl group, more preferably a $C_{8-14}$ alkyl group. Compounds of the invention may advantageously comprise blends of compounds having different

numbers of carbon atoms in the alkyl group. Preferred compounds of the invention include those having numbers of carbon atoms in the alkyl group within the range 8 to 14, e.g. alkyl *para*-xylene sulphonates wherein the alkyl group has the distribution 35%w $C_{8-10}$, 40%w $C_{11-12}$, and 25%w $C_{13-14}$.

The invention also relates to a process for the preparation of compounds according to the invention, comprising alkylating *meta*- and/or *para*-xylene and sulphonating the alkylxylene(s) thus formed, optionally followed by converting the alkylxylene sulphonic acid(s) thus formed into the desired salt(s). The alkylation may be carried out in any manner known for analogous compounds, e.g. by a Friedel-Crafts reaction using alkyl halides, alkanols, or alkenes, in the presence of a Lewis acid catalyst.

Reaction using a linear alkyl halide will result in the eventual compound of formula II having n' = 0. Alkylation with linear alpha-olefins, e.g. those available from the "SHELL" Higher Olefins Process ("SHELL" is a registered trade mark) is very advantageous, and leads to the compounds of formula II wherein n' is 1, 2 or 3. By using mixtures of such olefins with an appropriate chain length distribution, desired mixtures of end products may be obtained. Advantageous catalysts are, for example, hydrogen fluoride and activated clays (e.g. "Fulcat"). (registered trade mark).

Product purity may be improved by topping and tailing distillations, so that products containing more than 95% of mono-alkyl xylenes can be obtained without many problems.

The sulphonation can then be performed in a manner known for analogous compounds, e.g. by contacting with concentrated sulphuric acid and/or with sulphur trioxide. Conveniently the acid reaction product is then neutralized with an appropriate base, e.g. with sodium hydroxide to produce the sodium sulphonate salt.

The invention also provides a detergent composition, comprising a compound according to the invention in association with at least one diluent and/or builder. Such compositions may be formulated in known manner. Diluents (or fillers) are generally inorganic salts, acids and bases that do not contribute to the detergency. Non-surfactant compounds that augment the cleaning effect of a composition are called builders: usually organic additives which enhance the detergency, foaming power, emulsifying power or soil suspending effect. In addition other special purpose additives may be present, such as bleaching agents, brightening agents, bactericides or emollients. One or more additional surface active components may be included if desired.

Since the present alkylxylene suphonate compounds are very useful in compositions for enhanced oil recovery applications, the invention further provides an aqueous composition specifically therefor, containing 2.0—10%v of compounds according to the invention, 0.5 to 2.0%v of a $C_{2-6}$ aliphatic alcohol, 0.05 to 2.0%v of a co-surfactant, e.g. selected from alcohol ethoxylated, ethoxy sulphates or ethoxy sulphonates, and 0 to 2.0%v of a (carrier) oil.

The invention still further provides use of compounds of the invention as surfactants, and more specifically, a method of displacing an oil within a subterranean reservoir, which comprises injecting compounds or a composition according to the invention and subsequently injecting water, optionally containing a polymeric thickener.

The present compounds have an advantageous ability to form stable microemulsions of oil and water, which are believed to be advantageous with respect to ordinary solutions because of lower interfacial tensions and lower tendency for the surfactant to be retained by rock in oil-bearing formations.

The invention will be further understood from the following illustrative examples.

Example 1

Preparation of sodium alkylxylene sulphonates

A. Linear alpha-olefins of varying chain lengths from the "SHELL" Higher Olefins Process (SHOP) were reacted with ortho-, meta-, and para-xylenes in a stirred batch reactor provided with a condenser, at atmospheric pressure and a temperature of 138 to 145°C. The xylene/olefin molar ratios were in the range 5 to 10. As catalysts an activated clay was used, which is available commercially under the registered trade mark "FULCAT 22B". The progress of the reaction was followed by periodic gas-liquid chromatographic (GLC)-analysis. When after about five hours all the olefin had reacted, the catalyst was filtered off, after which the remaining unreacted xylene was flashed off in a rotating evaporator apparatus. Products containing 93—99% of mono-alkylxylenes were obtained via topping and tailing distillations.

The molar isomer percentages of monoalkyl-m-xylenes and monoalkyl-p-xylenes in the alkylation products from *meta*- and *para*-xylenes, having formula

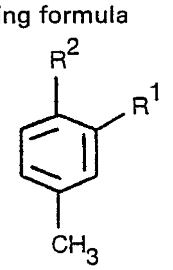

wherein one of $R^1$ and $R^2$ is methyl, the other being an alkyl group of formula

$$-CH(CH_2)_nCH_3$$
$$|$$
$$(CH_2)_{n'}H$$

where n' is 1, 2 or 3, were determined by $^{13}C$—NMR (nuclear magnetic resonance).

Results are given in Table I following.

TABLE I

| Starting materials | | Product | | | | | |
|---|---|---|---|---|---|---|---|
| | | | | %mol of isomer having n' | | | Total of |
| xylene | olefin | $R^1$ | $R^2$ | 1 | 2 | 3 | n'=1+2+3 |
| *meta* | $C_{8-10}$ | $CH_3$ | $C_{8-10}$alkyl | 33 | 24 | 19 | 76 |
| | $C_{11-12}$ | $CH_3$ | $C_{11-12}$alkyl | 26 | 20 | 15 | 61 |
| | $C_{13-14}$ | $CH_3$ | $C_{13-14}$alkyl | 21 | 19 | 14 | 54 |
| *para* | $C_{8-10}$ | $C_{8-10}$alkyl | $CH_3$ | 46 | 22 | 18 | 86 |
| | $C_{11-12}$ | $C_{11-12}$alkyl | $CH_3$ | 34 | 22 | 18 | 74 |
| | $C_{13-14}$ | $C_{13-14}$alkyl | $CH_3$ | 27 | 18 | 16 | 61 |

B. Alkylated xylenes and blends thereof from step A were sulphonated with gaseous sulphur trioxide in a stirred batch reactor. The ratios of the reactants and the other reaction conditions were as follows:

$SO_3$/alkylated xylene = 1.05 (mole/mole)
$SO_3$ in $N_2$ = 7%v
reaction temperature= 50°C

The $SO_3$ was added during 2.5 hours after which the reaction was allowed to proceed for yet another half hour at 50°C. After ageing for one day at ambient temperature, the sulphonated products were neutralized with a 46%w NaOH solution in water, at ambient temperature. Especially the para-xylene derived materials generated were extremely viscous pastes, and during the neutralization therefore water was added to lower the viscosity. The final products were off-white foams with a concentration of approximately 1.6 meq/g active matter, as determined by two-phase titration.

94% mol of the alkyl *meta*-xylene sulphonates was found to be 5-alkyl-2,4-dimethyl benzene sulphonate material, and 100% mol of the alkyl para-xylene sulphonates was found to be 4-alkyl-2,5-dimethyl benzene sulphonate material.

Example 2

Phase behaviour

Table II following lists some parameters of a surfactant-brine-oil system composed of the surfactant blends indicated, a Far Eastern crude oil, and a synthetic reservoir water. The surfactant consisted in all cases of sodium linear alkylxylene sulphonates, and was based on *meta-* or *para*-xylene, or — for comparison — on *ortho*-xylene. The surfactant was present in a concentration of 1 meq/18 ml of total system. The synthetic reservoir water (SRW) was made up of a NaCl and $CaCl_2$ solution, 100% SRW being set at 10.0 g/l NaCl and 0.0821 g/l $CaCl_2$, corresponding to a Na/Ca molar ratio of 231. The systems contained 0.6%w of sec. butanol as a co-surfactant in addition to the alkylxylene sulphonate surfactant. The midpoint salinity (MPS) is that concentration of salt expressed as % SRW in a brine (shortly often called brine concentration), that causes a (pseudo) three component system of surfactant-brine-oil to give a so-called type III phase behaviour, in which the (middle) micro-emulsion phase contains equal amounts of oil and brine. Among others the MPS is determined by the water solubility of the surfactant blend. For optimal results of micellar flooding it is considered of eminent importance to maintain type III phase behaviour during the full length of the flooding procedure. Consequently, besides the MPS, also the salinity window, which is the range of brine concentrations at which this type III phase behaviour is observed, is an important parameter. The salinity window (SW) is also expressed in % SRW.

The solubilization parameter (SP) at midpoint salinity,

$$\left(\frac{V_o}{V_s}\right)_{MPS},$$

is defined as the volume of oil (or brine) per volume (unit) of surfactant in the micro-emulsion phase. Obviously, the higher the SP the more efficient the surfactant is in solubilizing the oil. Under certain conditions there is a proportionality between the solubilization parameter and the interfacial tension (IFT) between the oil-micro-emulsion and microemulsion-brine phases at MPS. The higher the SP, the lower the IFT. This proportionality was confirmed, within experimental accuracy, for a few randomly chosen blends. All measurements were taken at a temperature of 67°C.

TABLE II

| Surfactant blend o, m or p | alkyl group weight fraction | | | MPS % SRW | SW % SRW | SP | IFT $\mu N.m^{-1}$ | Total %w of sulphonate of formula I having formula II where n'= 1, 2 or 3 |
|---|---|---|---|---|---|---|---|---|
| | $C_{8-10}$ | $C_{11-12}$ | $C_{13-14}$ | | | | | |
| **ortho* | 35 | 40 | 25 | 139 | 50 | 5.6 | 2 | — |
| **ortho* | 100 | 0 | 0 | 370 | 275 | 3.0 | 43 | — |
| *meta*[1)] | 35 | 40 | 25 | 413 | 180 | 7.2 | 3 | 61 |
| *para* | 35 | 40 | 25 | 670 | 170 | 9.2 | | 75 |
| *para* | 20 | 30 | 50 | 740 | 100 | 10.2 | | 70 |
| *para* | 10 | 20 | 70 | 500 | 55 | 12.0 | | 66 |
| *para* | 0 | 100 | 0 | 645 | 100 | 11.8 | | 74 |
| *para* | 0 | 0 | 100 | 350 | 70 | 11.0 | | 61 |
| 1 o/1 p[2)] | 35 | 40 | 25 | 365 | 120 | 7.4 | 2 | 37.5 |
| 1 o/3 p[2)] | 35 | 40 | 25 | 531 | 175 | 9.0 | | 56 |

* Comparative
[1)]containing only 0.33%w of s-butanol
[2)]containing only 0.28%w of s-butanol

It is clear that *meta*- and *para*-xylene based surfactant blends in accordance with the invention indeed have a higher water solubility than the corresponding *ortho*-xylene base blend, since in a surfactant-brine-oil system they reach MPS at considerably higher salinities. The maximum MPS to be obtained with a "pure" *ortho*-xylene based blend under the present experimental conditions is 370% SRW, but the related SP is very low. This is immediately reflected in a high interfacial tension. *Para*-xylene based blends have been found to exhibit advantageous properties. For instance, about the same MPS (365% SRW) can be obtained using a 1:1 mixture of *ortho*- and *para*-xylene based surfactants, whereas the SP is 7.4 and the IFT is correspondingly low.

**Claims**

1. Alkylxylene sulphonate compounds of formula

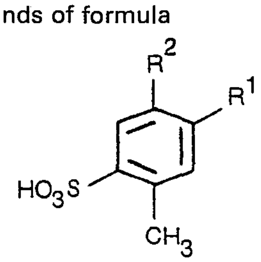

(I)

wherein one of $R^1$ and $R^2$ is a methyl group, the other being a $C_{6-20}$ alkyl group of formula

$$-CH(CH_2)_nCH_3 \quad (\text{with } (CH_2)_{n'}H \text{ attached to CH}) \qquad (II)$$

wherein n' is 0, 1, 2 or 3 and (n + n') is a number in the range 4 to 18; with the proviso that when both methyl groups are in the meta-position in relation to each other and when n' is 1, n is not equal to a number from 7 to 10; or in the form of a metal salt or an ammonium salt thereof.

2. Compounds according to claim 1 wherein $R^2$ represents a methyl group.

3. Compounds according to claims 1 to 2 in the form of the sodium salt thereof.

4. Compounds according to any of claims 1 to 3 wherein n' is 1, 2, or 3.

5. Process for the preparation of compounds as claimed in any of the preceding claims, comprising alkylating *meta-* and/or *para*-xylene and sulphonating the alkylxylene(s) thus formed, optionally followed by converting the alkylxylene sulphonic acid(s) thus formed into the desired salt(s).

6. A detergent composition comprising compounds as claimed in any of claims 1 to 4, in association with at least one diluent and/or builder.

7. An aqueous composition as claimed in claim 6, containing 2.0—10%v of compounds as claimed in any of claims 1 to 4, 0.5—2.0%v of a $C_{2-6}$ aliphatic alcohol, 0.05—2.0%v of a co-surfactant, and 0—2.0%v of an oil.

8. Method of displacing an oil within a subterranean reservoir which comprises injecting compounds as claimed in any of claims 1 to 4 or a composition as claimed in claim 6 or 7 and subsequently injecting water.

9. Use of compounds as claimed in any of claims 1 to 4 as a surfactant.

**Patentansprüche**

1. Alkylxylolsulfosäureverbindungen der Formel

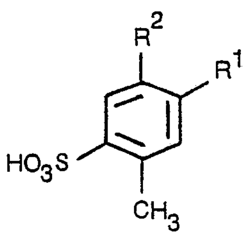

(I)

auch in Form eines Metallsalzes oder eines Ammoniumsalzes, in welcher Formel eine der Gruppen $R^1$ und $R^2$ eine Methylgruppe und die andere Gruppe eine $C_{6-20}$-Alkylgruppe der nachstehenden Formel darstellt:

$$-CH(CH_2)_nCH_3 \quad (\text{with } (CH_2)_{n'}H \text{ attached to CH}) \qquad (II)$$

in welcher n' den Wert 0, 1, 2 oder 3 hat und (n + n') eine Zahl im Bereich von 4 bis 18 ist, unter der Bedingung, daß, wenn beide Methylgruppen sich in Beziehung zueinander in der meta-Stellung befinden und wenn n' den Wert 1 hat, n nicht einer Zahl von 7 bis 10 entspricht.

2. Verbindungen gemäß Anspruch 1, in welchen $R^2$ eine Methylgruppe bedeutet.

3. Verbindungen gemäß Anspruch 1 oder 2 in Form ihres Natriumsalzes.

4. Verbindungen gemäß irgendeinem der Ansprüche 1 bis 3, in welchen n' den Wert 1, 2 oder 3 hat.

5. Verfahren zur Herstellung von Verbindungen wie in irgendeinem der vorhergehenden Ansprüche beansprucht, umfassend die Alkylierung von meta- und/oder para-Xylol und Sulfonierung des (der) so gebildeten Alkylxylols (Alkylxylole) und gegebenenfalls die Umwandlung des(der) so gebildeten Alkylxylolsulfosäure(n) in das(die) gewünschte(n) Salz(e).

6. Detergenzzusammensetzung umfassend Verbindungen, wie in irgendeinem der Ansprüche 1 bis 4 beansprucht, in Kombination mit mindestens einem Verdünnungsmittel und/oder einem Builder.

7. Eine wäßrige Zusammensetzung wie in Anspruch 6 beansprucht, enthaltend 2,0 bis 10 Volumenprozent an Verbindungen, wie in irgendeinem der Ansprüche 1 bis 4 beansprucht, 0,5 bis 2,0 Volumenprozent eines aliphatischen $C_{2-6}$-Alkohols, 0,05 bis 2,0 Volumenprozent eines Co-Tensids und 0 bis 2,0 Volumenprozent eines Öls.

8. Methode zum Verdrängen eines Öls innerhalb einer unterirdischen Lagerstätte, welche das Injizieren von Verbindungen wie in irgendeinem der Ansprüche 1 bis 4 beansprucht, oder einer Zusammensetzung wie in Anspruch 6 oder 7 beansprucht und anschließend das Injizieren von Wasser umfaßt.

9. Verwendung von Verbindungen wie in irgendeinem der Ansprüche 1 bis 4 beansprucht, als oberflächenaktives Mittel (Tensid).

**Revendications**

1. Des sulfonates d'alkylxylène de formule

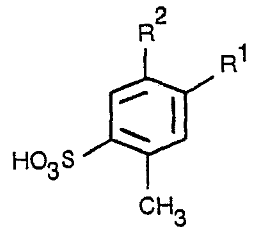

(I)

où l'un des groupes $R^1$ et $R^2$ est un groupe méthyle, l'autre étant un groupe alkyle en $C_{6-20}$ de formule

$$-\underset{\displaystyle |\atop (CH_2)_{n'}H}{CH}(CH_2)_nCH_3 \qquad (II)$$

où n' est 0, 1 ou 2 et (n + n') est un nombre de 4 à 18; avec la condition que quand les deux groupes méthyle sont dans la position méta l'un par rapport à l'autre et quand n' est 1, n ne doit pas être égal à un nombre de 7 à 10; ou sous la forme d'un sel de métal ou d'un sel d'ammonium d'un tel composé.

2. Des composés selon la revendication 1, dans lesquels $R^2$ représente un groupe méthyle.

3. Des composés selon les revendications 1 ou 2 sous la forme de leurs sels de sodium.

4. Des composés selon l'une quelconque des revendications 1 à 3, dans lesquels n' est 1, 2 ou 3.

5. Un procédé pour la préparation de composés tels que définis dans l'une quelconque des revendications précédentes, comprenant l'alkylation de *méta*- et/ou de *para*-xylène et la sulfonation du ou des alkylxylènes ainsi formés, cela étant suivi éventuellement d'une transformation de l'acide ou des acides alkylxylène sulfoniques ainsi formés de façon à donner le ou les sels désirés.

6. Une composition détergente comprenant des composés selon l'une quelconque des revendications 1 à 4 en association avec au moins un diluant et/ou un adjuvant.

7. Une composition aqueuse selon la revendication 6, contenant 2,0 à 10% en volume de composés selon l'une quelconque des revendications 1 à 4, 0,5 à 2,0% en volume d'un alcool aliphatique en $C_{2-6}$, 0,05 à 2,0% en volume d'un agent tensio-actif associé et 0 à 2,0% en volume d'une huile.

8. Une méthode de déplacement d'une huile dans un réservoir souterrain, qui comprend l'injection de composés selon l'une quelconque des revendications 1 à 4 ou d'une composition selon la revendication 6 ou 7 et ensuite l'injection d'eau.

9. L'utilisation de composés selon l'une quelconque des revendications 1 à 4 comme agent tensio-actif.